# EUROPÄISCHE PATENTSCHRIFT

(11) **EP 3 468 541 B1**
(45) Veröffentlichungstag und Bekanntmachung des Hinweises auf die Patenterteilung: **22.09.2021**
(21) Anmeldenummer: 17730120.7
(22) Anmeldetag: 08.06.2017
(51) Int. Cl.: A61K 31/035, A61P 31/00, A61P 31/04

(54) **WIRKSTOFFE GEGEN PROTOZOEN**
ANTIPROTOZOAL AGENTS
MATIERES CONTRE LES PROTOZOAIRES

(30) Priorität: 09.06.2016 EP 16173828
(43) Veröffentlichungstag der Anmeldung: 17.04.2019
(73) Patentinhaber: Christian-Albrechts-Universität zu Kiel, 24118 Kiel (DE)
(72) Erfinder: BEITZ, Eric, 24229 Dänischenhagen (DE); GOLLDACK, André, 24143 Kiel (DE); HENKE, Björn, 24116 Kiel (DE)
(74) Vertreter: Taruttis, Stefan Georg
(86) Internationale Anmeldenummer: PCT/EP2017/064026
(87) Internationale Veröffentlichungsnummer: WO 2017/211988

(56) Entgegenhaltungen:
- WO-A2-2013/048584
- FLECK LAURA E ET AL: "A screen for and validation of prodrug antimicrobials.", ANTIMICROBIAL AGENTS AND CHEMOTHERAPY 2014, Bd. 58, Nr. 3, 2014, Seiten 1410-1419, XP002764007, ISSN: 1098-6596

### SEQUENCE LISTING

<110> Christian-Albrechts-Universität zu Kiel
<120> Wirkstoffe gegen Protozoen
<130> K1027PCT
<150> EP16173828.1
   <151> 2016-06-09
<160> 6
<170> BiSSAP 1.3.6
<210> 1
   <211> 309
   <212> PRT
   <213> Plasmodium falciparum
<220>
   <223> PfFNT_PF3D7_0316600_PlasmoDB
<400> 1
   Met Pro Pro Asn Asn Ser Lys Tyr Val Leu Asp Pro Val Ser Ile Lys
   1               5                   10                  15
   Ser Val Cys Gly Gly Glu Glu Ser Tyr Ile Arg Cys Val Glu Tyr Gly
               20                  25                  30
   Lys Lys Lys Ala His Tyr Ser Asn Leu Asn Leu Leu Ala Lys Ala Ile
           35                  40                  45
   Leu Ala Gly Met Phe Val Gly Leu Cys Ala His Ala Ser Gly Ile Ala
       50                  55                  60
   Gly Gly Leu Phe Tyr Tyr His Lys Leu Arg Glu Ile Val Gly Ala Ser
   65                  70                  75                  80
   Met Ser Val Phe Val Tyr Gly Phe Thr Phe Pro Ile Ala Phe Met Cys
                   85                  90                  95
   Ile Ile Cys Thr Gly Ser Asp Leu Phe Thr Gly Asn Thr Leu Ala Val
               100                 105                 110
   Thr Met Ala Leu Tyr Glu Lys Lys Val Lys Leu Leu Asp Tyr Leu Arg
           115                 120                 125
   Val Met Thr Ile Ser Leu Phe Gly Asn Tyr Val Gly Ala Val Ser Phe
       130                 135                 140
   Ala Phe Phe Val Ser Tyr Leu Ser Gly Ala Phe Thr Asn Val His Ala
   145                 150                 155                 160
   Val Glu Lys Asn His Phe Phe Gln Phe Leu Asn Asp Ile Ala Glu Lys
                   165                 170                 175
   Lys Val His His Thr Phe Val Glu Cys Val Ser Leu Ala Val Gly Cys
               180                 185                 190
   Asn Ile Phe Val Cys Leu Ala Val Tyr Phe Val Leu Thr Leu Lys Asp
           195                 200                 205
   Gly Ala Gly Tyr Val Phe Ser Val Phe Phe Ala Val Tyr Ala Phe Ala
       210                 215                 220
   Ile Ala Gly Tyr Glu His Ile Ile Ala Asn Ile Tyr Thr Leu Asn Ile
   225                 230                 235                 240
   Ala Leu Met Val Asn Thr Lys Ile Thr Val Tyr Gln Ala Tyr Ile Lys
                   245                 250                 255
   Asn Leu Leu Pro Thr Leu Leu Gly Asn Tyr Ile Ala Gly Ala Ile Val
               260                 265                 270
   Leu Gly Leu Pro Leu Tyr Phe Ile Tyr Lys Glu His Tyr Tyr Asn Phe
           275                 280                 285
   Glu Arg Ser Lys Arg Asp Asn Asn Asp Ala Gln Met Lys Ser Leu Ser
       290                 295                 300
   Ile Glu Leu Arg Asn
   305
<210> 2
   <211> 412
   <212> PRT
   <213> Toxoplasma gondii
<220>
   <223> TgFNT1_TGGT1_209800_ToxoDB
<400> 2
   Met Val Val Thr Ala Ser Pro Asp Thr Tyr Leu His Val Ile Asp Tyr
   1               5                   10                  15
   Gly Leu Lys Lys Val Arg Leu Arg Phe Asp Arg Leu Leu Leu Gln Ala
               20                  25                  30
   Phe Met Ala Gly Val Tyr Ile Gly Met Ala Gly Asn Ala Cys Ile Ser
           35                  40                  45
   Leu Ala Gly Gly Phe Ser Thr Asp Pro Ala Asp Pro Lys Ala Ile Thr
       50                  55                  60
   Ala Gly Val Gln Lys Phe Ile Tyr Ala Ser Ile Phe Pro Val Ala Phe
   65                  70                  75                  80
   Ile Ala Ile Ile Met Thr Gly Ala Glu Leu Phe Thr Gly Asn Thr Met
                   85                  90                  95
   Thr Met Leu Ile Cys Trp Phe Glu Arg Arg Ile Thr Ile Trp Gln Leu
               100                 105                 110
   Leu Gln Asn Trp Ala Gly Ser Phe Leu Gly Asn Trp Leu Gly Thr Met
           115                 120                 125
   Phe Ser Ala Tyr Phe Leu Thr Tyr Leu Cys Cys Pro Phe Asp His Asp
       130                 135                 140
   Pro Tyr Leu Ser Tyr Leu Asn Tyr Thr Ala Ala Ser Lys Val Ser Tyr
   145                 150                 155                 160
   Gly Trp Gly Ser Cys Phe Leu Arg Gly Val Gly Cys Asn Thr Trp Val
                   165                 170                 175
   Cys Leu Ala Val Trp Phe Val Val Ala Cys Asp Asp Ala Ala Gly Lys
               180                 185                 190
   Ile Leu Ala Leu Trp Phe Pro Ile Val Ala Phe Val Leu Ser Ser Tyr
           195                 200                 205
   Glu His Ile Ile Ala Asn Leu Tyr Thr Leu Gln Leu Cys Ala Met Leu
       210                 215                 220
   Gly Val Asp Thr Ser Leu Ala Asp Met Ile Ala Phe Asn Leu Leu Pro
   225                 230                 235                 240
   Thr Leu Leu Gly Asn Leu Phe Gly Gly Cys Gly Leu Ile Gly Met Val
                   245                 250                 255
   Tyr Phe Tyr Asn Phe Tyr Pro Val Val Gly His Gly Asp Asp Ala Ala
               260                 265                 270
   Glu Gly Ser Ile Cys Gly Ser Ser Glu Lys Glu Glu Cys Pro Ser Leu
           275                 280                 285
   Val Gly Val Pro Arg Gly Ala Ser Val Asn Ser Leu Ala Val Ser Ala
       290                 295                 300
   Ile Pro Ser Val Phe Ser Ala Pro Arg Gly Gln Arg Glu Ser Phe Ala
   305                 310                 315                 320
   Gly Glu Ser Ser Thr Leu Val Met Gly Asp Ile Lys Arg Gln Arg Ser
                   325                 330                 335
   Met Ala Ser Thr Arg Lys Leu Gly Gly Gly Ala Asp Lys Lys Asp Val
               340                 345                 350
   Gln Leu Thr Val Arg Gln Phe Asp Glu Thr Glu Met Gln Ser Thr Met
           355                 360                 365
   Glu Asp Met Phe Gly Leu Glu Asp Pro Arg Asn Ala Pro Lys Gly Asn
       370                 375                 380
   Pro Gly Thr Asn Pro Pro Ser Lys Ser Pro Glu Ser Ser Ala Thr Gly
   385                 390                 395                 400
   Gly Thr Gly Ala Ala Ala Ser Pro Thr Ala Thr Ser
                   405                 410
<210> 3
   <211> 463
   <212> PRT
   <213> Toxoplasma gondii
<220>
   <223> TgFNT3_TGGT1_292110_ToxoDB
<400> 3
   Met Cys Ser Ile Pro Pro Leu Arg Leu Leu Glu Val Val Asp Asp Leu
   1               5                   10                  15
   Thr Cys Leu Val Asn Ser Ser Gln Tyr Ser Arg Trp Asp Phe Phe Thr
               20                  25                  30
   Phe Thr Leu Pro Gly Ser Thr Leu Pro Met Arg Lys Glu Phe Thr Ser
           35                  40                  45
   Ser Cys Lys Gly Ser Phe Ser Asn His Pro Val Pro Asp His Pro Cys
       50                  55                  60
   Lys Leu Val Val Phe Val Arg Pro Lys Met Val Val Thr Ala Gly Ala
   65                  70                  75                  80
   Asp Ala Tyr Leu Lys Ile Leu Glu Tyr Gly Val Lys Lys Thr Gln Leu
                   85                  90                  95
   Arg Ile Asp Arg Leu Leu Leu Gln Ala Phe Met Ala Gly Ile Phe Val
               100                 105                 110
   Ala Met Ala Gly His Cys Cys Thr Val Leu Ala Gly Ser Tyr Pro Thr
           115                 120                 125
   Asp Pro Gly Asp Pro Leu Ala Val Ala Lys Pro Thr Gln Lys Phe Ile
       130                 135                 140
   Tyr Gly Ala Leu Phe Pro Val Ala Phe Ile Cys Ile Ile Leu Thr Gly
   145                 150                 155                 160
   Ala Glu Leu Phe Thr Gly Asn Thr Met Thr Met Leu Ile Cys Tyr Phe
                   165                 170                 175
   Gln Lys Arg Val Thr Met Leu Gln Leu Gly Val Asn Trp Leu Gly Ser
               180                 185                 190
   Leu Ala Gly Asn Trp Leu Gly Ala Leu Phe Gly Ala Tyr Phe Leu Ser
           195                 200                 205
   Tyr Leu Thr Gly Ala Leu Gly Asp Glu His Val Arg Gln Phe Leu Phe
       210                 215                 220
   Arg Thr Cys Val Asn Lys Ile Ser Tyr Gly Trp Gly Glu Cys Phe Leu
   225                 230                 235                 240
   Arg Gly Val Gly Cys Asn Thr Phe Val Cys Leu Ala Val Trp Ala Val
                   245                 250                 255
   Ile Ala Ser Glu Asn Val Ala Gly Lys Val Leu Val Met Trp Phe Pro
               260                 265                 270
   Ile Val Ala Phe Cys Val Gly Gly Tyr Glu His Ile Ile Ala Asn Met
           275                 280                 285
   Tyr Thr Leu Gln Ala Gly Leu Met Ala Gly Ala Pro Val Ala Ile Leu
       290                 295                 300
   Asp Val Ile Ala Phe Asn Phe Leu Pro Thr Leu Leu Gly Asn Ile Val
   305                 310                 315                 320
   Gly Gly Cys Leu Leu Val Gly Ala Val Tyr Ala Tyr Asn Phe Tyr Pro
                   325                 330                 335
   Thr Leu Ser Tyr Thr Glu Thr Thr Gly Ala Lys Val Tyr Val Gln Glu
               340                 345                 350
   Val Gly Pro Val Leu Asp Arg Arg Ser Ser Met Gln Val Ser Met Thr
           355                 360                 365
   Glu Arg Glu Pro Asp Gly Gln Val Val Thr Glu Tyr Glu Ala Val Pro
       370                 375                 380
   Phe Glu Ser Phe Gly Gly Glu Tyr Ile Val Asn Lys His Ala Thr Met
   385                 390                 395                 400
   Ala Ala Pro Ile Pro Ser Arg Ala Ser Ser Phe Leu Tyr Pro Phe Gln
                   405                 410                 415
   Trp Gln Arg Gln Arg Ser Gln Ser Gly Asn Leu Ser Thr His Ala Arg
               420                 425                 430
   Leu Asp Leu Pro Asn Arg Pro Val Glu Pro Pro Ser Asp Gly Leu Glu
           435                 440                 445
   Val Thr Pro Gln Ser Gln Thr Ala Glu Ser Val Ala Gln Gln Val
       450                 455                 460
<210> 4
   <211> 501
   <212> PRT
   <213> Toxoplasma gondii
<220>
   <223> TgFNT2_TGGT1_229170_ToxoDB
<400> 4
   Met Val Leu Ala Ala Ser Pro Glu Ala Tyr Arg Lys Val Ile Glu Tyr
   1               5                   10                  15
   Gly Ile Lys Lys Thr Lys Leu Arg Ile Asp Arg Leu Phe Leu Gln Ala
               20                  25                  30
   Ile Met Ala Gly Ile Tyr Val Gly Met Ala Gly His Ala Cys Thr Ala
           35                  40                  45
   Leu Ala Gly Ala Tyr Ser Thr Asp Pro Ala Asn Pro Leu Ala Val Ser
       50                  55                  60
   Lys Ala Thr Gln Lys Phe Leu Tyr Ala Ser Leu Phe Pro Val Ala Phe
   65                  70                  75                  80
   Ile Ala Ile Ile Phe Thr Gly Ala Glu Leu Phe Thr Gly Asn Thr Met
                   85                  90                  95
   Thr Met Leu Val Cys Leu Leu Glu Arg Arg Val Thr Ala Leu Gln Leu
               100                 105                 110
   Cys Ile Asn Trp Ile Cys Ser Leu Val Gly Asn Trp Ala Gly Ala Leu
           115                 120                 125
   Phe Ala Ala Tyr Phe Leu Ser Tyr Leu Pro Gly Val Leu Gln Asp Pro
       130                 135                 140
   Asp His Leu His Tyr Leu Glu Asp Val Ala Ala His Lys Thr Glu Leu
   145                 150                 155                 160
   Ser Phe Leu Gln Cys Phe Cys Leu Ala Val Gly Cys Asn Thr Phe Val
                   165                 170                 175
   Cys Leu Ala Val Trp Phe Val Ile Ala Ser Asp Asp Ala Ala Gly Lys
               180                 185                 190
   Ile Met Ser Met Trp Phe Pro Ile Val Ser Phe Cys Val Ala Gly Tyr
           195                 200                 205
   Glu His Ile Ile Ala Asn Phe Tyr Thr Leu Gln Cys Ala Leu Met His
       210                 215                 220
   Gly Val Gly Pro Gly Val Gly Thr Val Ile Leu Lys Asn Phe Ile Pro
   225                 230                 235                 240
   Thr Leu Leu Gly Asn Ile Val Gly Gly Cys Gly Leu Val Gly Ala Val
                   245                 250                 255
   Tyr Trp Tyr Asn Phe Tyr Pro Thr Val Cys Val Val Gln Glu Ala Arg
               260                 265                 270
   Gln Pro Leu Pro Leu Ser Glu Asn Ala Pro Ser Ser Thr Arg Gln Val
           275                 280                 285
   Val Ala Asp Leu Phe Ser Leu Trp Gly Arg Glu Ser Ser Thr Pro Gly
       290                 295                 300
   Val Ser Ala Ser Pro Pro Asp Ala Ala Thr Asn Ala Gly Cys Ser Ala
   305                 310                 315                 320
   Leu Asp Pro Pro Arg Asn Ala Leu Leu Ala Ala Gly Lys Asn Phe Gly
                   325                 330                 335
   Asn Leu Ser Ala Gly Asp Arg Gly Ala Leu Ala Glu Gly Ile Pro Gly
               340                 345                 350
   Gly Ala Cys Glu Asp Cys Leu Leu Val Pro Arg Ala Ser Phe Gly Gly
           355                 360                 365
   Glu Tyr His Pro Pro Gln Gln Gly Asp Ala Gly Arg Trp Cys Lys Pro
       370                 375                 380
   Ser Lys Ala Ala Val Gly Ser Gly Gly Val Leu Cys His Val Gln Ser
   385                 390                 395                 400
   Pro Ala Ala Leu Glu Ala Val Ser Asn Ser Pro Leu Arg Glu Asn Ser
                   405                 410                 415
   Gly Val Pro Ser Gly Gly Leu Leu Leu Cys Glu Gly Arg Val Arg Arg
               420                 425                 430
   Ser Ser Arg Glu Arg Glu Pro Glu Arg Gly Gly Glu Glu Glu Glu Gly
           435                 440                 445
   Ala Ser Pro Glu Glu Glu His Pro Ala Val Thr Leu Ser Ile Pro Pro
       450                 455                 460
   Thr Asp Phe His Pro His Val Pro Arg Glu Val Glu Gln Ser Ser Leu
   465                 470                 475                 480
   Leu Glu Glu Thr Arg Val Ala Ala Glu Asn Ser Ala Leu Glu Glu His
                   485                 490                 495
   Pro Ala Ser Thr Ile
               500
<210> 5
   <211> 356
   <212> PRT
   <213> Entamoeba histolytica
<220>
   <223> EhFNT_EHI_198990_AmoebaDB
<400> 5
   Met Pro Arg Glu Lys Pro Arg Ala Asp Glu Ile Ala Ile Glu Met Met
   1 5 10 15
   Ser Val Cys Glu Asp Glu Thr Glu Val Glu Gln Asp Pro Arg Glu Leu
   20 25 30
   Tyr Glu Glu Glu Met Lys Glu Gln Gln Gln Ile Asp Cys Ser Lys Gln
   35 40 45
   Gln Lys Glu Val Val Ala Ile Glu Glu Leu Glu Lys Arg Asn Ile Asn
   50 55 60
   Lys His Phe Phe Ser Ile Gln Pro Asn Thr Gln Ile Pro Val Ile Ser
   65 70 75 80
   Ser Asn Tyr Ile Ala Pro Val Asp Thr Ser Arg Leu Leu Val Leu Ile
   85 90 95
   Gly Lys Thr Lys Ala Thr Tyr Pro Ile Met Lys Met Phe Ser Leu Ser
   100 105 110
   Val Leu Ala Gly Met Leu Leu Ser Val Gly Gly Leu Leu Ser Ile Thr
   115 120 125
   Ile Gly Lys Gly Ile Pro Ser Ser Asp Ile Gly Ile Gln Lys Ile Val
   130 135 140
   Phe Gly Phe Phe Asn Ser Val Gly Leu Asn Leu Val Val Leu Cys Gly
   145 150 155 160
   Gly Glu Leu Phe Thr Ser Asn Cys Ala Phe Leu Ile Pro Gly Phe Met
   165 170 175
   Glu Gly Ala Tyr Ser Arg Trp Leu Phe Phe Lys Thr His Phe Val Val
   180 185 190
   Tyr Phe Gly Asn Leu Val Gly Ser Ile Phe Val Ser Thr Tyr Phe Gly
   195 200 205
   Lys Leu Leu Gly Ser Phe Glu Ser Pro Met Tyr Leu Ser Ala Val Lys
   210 215 220
   Gln Ile Gly Glu Thr Lys Val Ala Met Asn Trp Gly Arg Ala Leu Leu
   225 230 235 240
   Ser Gly Ile Gly Cys Asn Trp Leu Val Cys Cys Ala Val Tyr Phe Ser
   245 250 255
   Ala Ser Ala Lys Asp Leu Leu Ser Lys Leu Val Val Ile Ser Phe Leu
   260 265 270
   Val Leu Thr Phe Ala Ser Leu Glu Phe Glu Asn Cys Val Gly Asn Met
   275 280 285
   Phe Leu Leu Ser Leu Ser His Met Tyr Gly Gly Asn Phe Thr Leu Gly
   290 295 300
   Gln Trp Ile Leu Asn Asn Leu Ile Pro Val Ser Ile Gly Asn Phe Ile
   305 310 315 320
   Gly Gly Thr Phe Leu Leu Gly Ile Pro Leu Trp Tyr Val His Val Ser
   325 330 335
   Asn Val Tyr Asn Ile Pro Phe Leu Asp Pro Leu Tyr Gln Gln Ser Gln
   340 345 350
   Ala Lys Thr Gln
   355
<210> 6
   <211> 269
   <212> PRT
   <213> Salmonella enterica subsp. enterica serovar Typhimurium
<220>
   <223> StNirC_AAA27040_NCBI
<400> 6
   Met Phe Thr Asp Ser Ile Asn Lys Cys Ala Ala Lys Leu Arg Ala Ser
   1               5                   10                  15
   Ala Pro Val Ser Ala Asn Asn Pro Leu Gly Phe Trp Val Ser Ser Ala
               20                  25                  30
   Met Ala Gly Ala Tyr Val Gly Leu Gly Ile Ile Leu Ile Phe Thr Leu
           35                  40                  45
   Gly Asn Leu Leu Asp Pro Ser Val Arg Pro Leu Val Met Gly Ala Thr
       50                  55                  60
   Phe Gly Ile Ala Leu Thr Leu Val Ile Ile Ala Gly Ser Glu Leu Phe
   65                  70                  75                  80
   Thr Gly His Thr Met Phe Leu Thr Leu Gly Val Lys Ala Gly Thr Ile
                   85                  90                  95
   Ser His Gly Gln Met Trp Ala Ile Leu Pro Gln Thr Trp Leu Gly Asn
               100                 105                 110
   Leu Val Gly Ser Val Phe Val Ala Leu Leu Tyr Ser Trp Gly Gly Gly
           115                 120                 125
   Ser Leu Leu Pro Val Asp Thr Ser Ile Val His Ser Val Ala Leu Ala
       130                 135                 140
   Lys Thr Thr Ala Pro Ala Thr Val Leu Phe Phe Lys Gly Ala Leu Cys
   145                 150                 155                 160
   Asn Trp Leu Val Cys Leu Ala Ile Trp Met Ala Ile Arg Thr Glu Gly
                   165                 170                 175
   Thr Ala Lys Phe Leu Ala Ile Trp Trp Cys Leu Leu Ala Phe Ile Ala
               180                 185                 190
   Ser Gly Tyr Glu His Ser Val Ala Asn Met Thr Leu Phe Ala Leu Ser
           195                 200                 205
   Trp Phe Gly His His Ser Asp Ala Tyr Thr Leu Ala Gly Ile Gly His
       210                 215                 220
   Asn Leu Leu Trp Val Thr Leu Gly Asn Thr Leu Ser Gly Val Val Phe
   225                 230                 235                 240
   Met Gly Leu Gly Tyr Trp Tyr Ala Thr Pro Lys Ser Glu Arg Pro Ala
                   245                 250                 255
   Pro Ala Lys Ile Asn Gln Pro Glu Ala Ala Ala Asn Asn
               260                 265

## Beschreibung

Die vorliegende Erfindung betrifft Verbindungen, die gegen Protozoen gerichtet sind und insbesondere die Verbindungen zur Verwendung bei der Behandlung von Infektionen durch Protozoen. Des Weiteren betrifft die Erfindung ein Verfahren zur Hemmung, bevorzugt Zerstörung, von Protozoen durch Kontaktieren von Protozoen mit zumindest einer der Verbindungen. Die Verbindungen zeichnen sich dadurch aus, dass sie den Formiat-Nitrit-Transporter von Protozoen hemmen.

### Stand der Technik

Die EP 2 483 274 B1 beschreibt eine Vielzahl von Wirkstoffen gegen Malaria, die die Dihydroorotatdehydrogenase von Plasmodium hemmen sollen.

Die EP 2 526 090 B1 beschreibt Aminopyridinderivate als pharmazeutischen Wirkstoff insbesondere gegen Malaria.

Fleck et al., Antimicrobial Agents and Chemotherapy, 1410-1419 (2014), beschreiben Verbindungen mit einem Trifluormethylrest als Wirkstoff gegen *E*. *coli.*

Die WO2013/048584 A2 beschreibt eine Verbindung, die unter die hiesige Struktur I fällt, als Wirkstoff gegen Bakterien.

Die Organisation Medicines for Malaria Venture (MMV) beschreibt als Wirkstoffe gegen Malaria unter insgesamt ca. 400 anderen Verbindungen die folgenden:

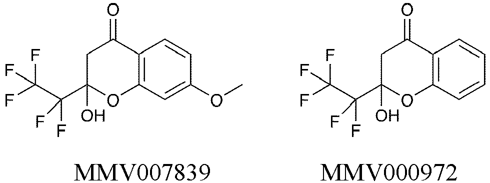

### Aufgabe der Erfindung

Die Aufgabe der Erfindung liegt in der Bereitstellung von alternativen Verbindungen, die als Wirkstoffe insbesondere gegen Protozoen wirksam sind. Bevorzugt sind die alternativen Verbindungen gegen ein anderes Zielmolekül von Protozoen wirksam als es bislang bekannt ist.

### Beschreibung der Erfindung

Die Erfindung löst die Aufgabe mit den Merkmalen der Ansprüche, insbesondere mittels einer Verbindung und deren Derivaten zur Verwendung gegen Protozoen bzw. zur Behandlung einer Infektion mit Protozoen, wobei die Verbindung die folgende Struktur I aufweist oder daraus besteht, in der der Rest R1 ein Perfluoralkyl ist, bei dem das Alkyl ein gradkettiges oder verzweigtes C₁- bis C₄-alkyl ist: 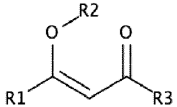 Struktur I, mit
R1 = Perfluor-C₂- bis C₄-alkyl, gradkettig oder verzweigt, insbesondere, Pentafluorethyl, Heptafluorpropyl, Nonafluorbutyl, davon bevorzugt Pentafluorethyl, einschließlich Solvaten und Salzen dieser.

R2 ist H oder ein C₁- bis C₁₂-Alkyl, z.B. Methyl, Ethyl, *n*-Propyl, *n*-Butyl, *n*-Pentyl, *n*-Hexyl, *n*-Octyl, *n*-Decyl, *n*-Dodecyl, *iso*-Propyl, *iso*-Butyl, tert-Butyl, 2,2-Dimethylpropyl oder Cyclohexyl, davon bevorzugt H oder Ethyl, oder R2 ist eine Carbonylgruppe mit H oder einem C₁- bis C₁₂-Alkyl, das z.B. Methyl, Ethyl, *n*-Propyl, *n*-Butyl, *n*-Pentyl, *n*-Hexyl, *n-*Octyl, *n*-Decyl, *n*-Dodecyl, *iso*-Propyl, *iso*-Butyl, *tert*-Butyl, 2,2-Dimethylpropyl oder Cyclohexyl ist. Auf Grundlage der derzeitigen Analysen ist anzunehmen, dass das wirksame Pharmakophor von Struktur I mit R1 gebildet wird.

R3 ist ein Aromat, ein aromatischer 5-Ring oder ein aromatischer 6-Ring, der optional daran kondensierte aromatische Ringe aufweisen kann. Der Aromat ist direkt oder über ein C₁- bis C₃-Alkyl gebunden. Eine Hydroxylgruppe in R3 im Abstand von zwei Kohlenstoffen zum Carbonyl-Kohlenstoff der Struktur I führt zur Hemiketalbildung wie in MMV007839; das Hemiketal stellt ein gut membrangängiges internes Prodrug dar. Z.B. ist R3 aus der Gruppe ausgewählt, die Aromaten, die in para- oder meta-Stellung zur Struktur I mit einem gradkettigen oder verzweigten C₁- bis C₁₂-Alkylrest, C₁- bis C₁₂-Alkyloxyrest oder Halogen substituiert sind, insbesondere die folgenden Reste 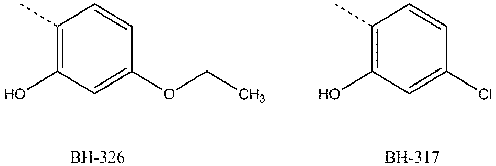 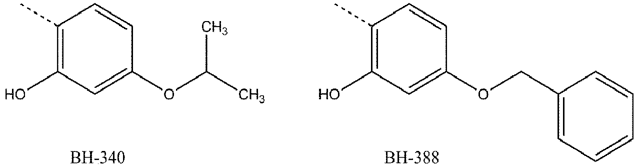 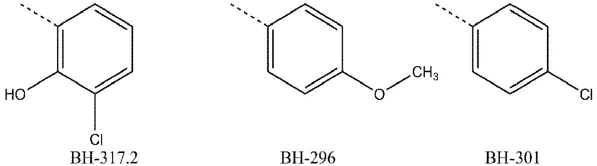 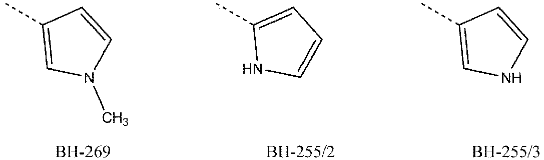 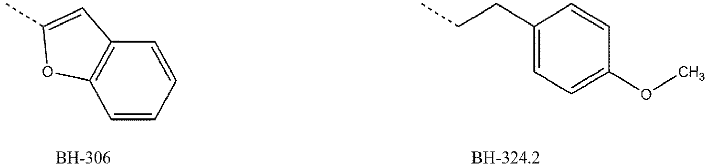 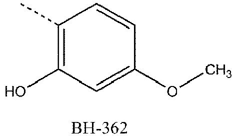 umfasst oder daraus besteht. Die Bezeichnungen unter den Resten R3 nennen die Namen der Verbindungen aus Struktur I mit R1 = Perfluor-C₂- bis C₄-alkyl, bevorzugt Pentafluorethyl, R2 = H oder Ethyl, bevorzugt H und dem angegeben Rest für R3.

Der Aromat kann ein Phenyl, ein Pyrrolyl, Furyl, Pyridyl oder Benzofuryl sein.

Bevorzugt weist R3 einen Phenylring auf, der im Abstand von zwei Kohlenstoffen zum Carbonyl-C-Atom der Struktur I, entsprechend in ortho-Stellung zur Ketogruppe, eine Hydroxylgruppe aufweist und in para-Stellung zur Ketogruppe ein Halogenatom, insbesondere Cl, oder einen Alkoxyrest, z.B. eine Methoxygruppe, bevorzugt eine Ethoxy-oder Propoxygruppe aufweist, oder besteht daraus. Die Propoxygruppe kann eine n-Propoxygruppe oder eine Isopropoxygruppe sein.

Bevorzugte Verbindungen, in denen R3 ein Phenylrest ist, der in ortho-Stellung zur Ketogruppe eine Hydroxylgruppe aufweist und in para-Stellung zur Ketogruppe ein Cl oder eine Ethoxy- oder Propoxygruppe, sind z. B. die Verbindungen BH-326, BH-317 und BH-340.

Erfindungsgemäß ist die offenkettige vinyloge Carbonsäure die aktive, substratähnliche Wirkform, während die Hemiketale MMV007839 und MMV0972 reversible, interne Prodrugs mit guter Resorption in Malaria-Parasiten darstellen. Die Nutzung des Prodrug-Prinzips durch Einführung einer Hydroxylgruppe in R3 kann somit die Verfügbarkeit im Parasiten erhöhen.

Optional ist die Verbindung MMV007839 und/oder die Verbindung MMV000972 und/oder die offenkettige vinyloge Carbonylverbindung, die mit der Verbindung MMV007839 und/oder der Verbindung MMV000972 im Gleichgewicht steht oder deren Isomere bzw. Hemiketalisomere sind, von den Verbindungen der Struktur I ausgenommen.

Diese Verbindungen zeichnen sich dadurch aus, dass sie das Formiat-Nitrit-Transporterprotein (FNT) aus Protozoen hemmen. Der Mensch besitzt diese Formiat-Nitrit-Transporterproteine nicht. Der humane Laktat-Transporter wird durch die Verbindungen wesentlich schlechter gehemmt. Die Hemmung des FNT führt dazu, dass in den Protozoen Acetat und Laktat, das z.B. bei der anaeroben Verstoffwechselung von Glucose aus Pyruvat entsteht, nicht effektiv transportiert wird, so dass die Zellen z.B. übersäuern und absterben.

Generell weisen nur Protozoen, gegen die die Verbindungen als Wirkstoff verwendet werden, Formiat-Nitrit-Transporterproteine (FNT) auf. Zur Anwendung der Verbindungen bevorzugte Protozoen sind einzellige Parasiten von Mensch und Tier, insbesondere Plasmodium falciparum (Pf; PfFNT, Sequence-ID PF3D7_0316600 PlasmoDB) und Plasmodium vivax (Pv; PvFNT, Sequence-ID PVX_095405 PlasmoDB), zwei Erreger der Malaria, Toxoplasma gondii (Tg; TgFNT1, Sequence-ID TGGT1_209800 ToxoDB; TgFNT2, Sequence-ID TGGT1_292110 ToxoDB; TgFNT3, Sequence-ID TGGT1_229170 ToxoDB), der Erreger der Toxoplasmose, Entamoeba histolytica (Eh; EhFNT, Sequence-ID EHI_198990 AmoebaDB), der Erreger der Amöbenruhr.

Die Erfindung wird nun genauer anhand von Beispielen erläutert.

### Beispiel 1: Wirkung von Verbindungen gegen FNT

Die Wirkung der folgenden Verbindungen auf das Wachstum von Plasmodium falciparum wurde anhand kultivierter Plasmodien getestet:

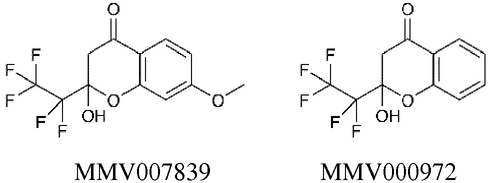

Die Hemmwirkung IC50 auf das Wachstum der Plasmodiumkultur wurde für MMV007839 zu 140 nM bestimmt, für MMV000972 zu 1,7 µM. Die Plasmodiumkultur wurde in 5% 0+ Erythrocyten in RPMI 1640 Medium mit 0,5% Albumax bei 37°C geführt. Die IC50 wurde 48 h nach Zugabe der Substanz-Verdünnungen über Auszählung der Parasitämie per FACS bestimmt. Nach 24 h erfolgte ein Mediumwechsel und die Verbindungen wurden frisch zugegeben.

Bei der Behandlung der kultivierten Plasmodien mit MMV007839 bei einer Konzentration entsprechend der dreifachen IC50-Konzentration wurden resistente Plasmodien erhalten, deren IC50 sich zu 35 µM verschoben hatte. Eine Sequenzierung des FNT-Gens der resistenten Plasmodien ergab eine Mutation Gly107Ser auf Proteinebene.

Mit Ausnahme der Verbindung BH-296 ist bislang keine Verbindung bekannt, die im pharmazeutisch akzeptablen Konzentrationsbereich auch gegen die in Beispiel 1 beschriebenen resistenten Parasiten wirksam ist.

Der FNT des Plasmodium-Wildtyps (erhältlich unter Zugangsnr. PF3D7_0316600 bei der Datenbank PlasmoDB) und der resistenten Plasmodien (Position der Mutation im offenen Leserahmen: Guanin 319 zu Adenin) wurden in der Hefe Saccharomyces cerevisiae W303-1A jen1Δ ady2Δ vom Plasmid pDR196 (PMA Promotor) exprimiert. In der Hefe waren die endogenen Gene für die Monocarboxylat-Transporter Jen1p (Sequence-Accession-No. CAA82062 NCBI) und Ady2p (Sequence-Accession-No. KZV12856) deletiert (bezogen von M. Casal Universidade do Minho, Portugal).

Diese Hefe wurde in SD Medium unter Zusatz von Adenin, Histidin, Leucin, Tryptophan und 2% (wt/V) Glucose bei 30°C bis zu einer OD₆₀₀ von 0,8 bis 1,0 wachsen gelassen, durch Zentrifugieren geerntet und einmal mit Wasser gewaschen und erneut abzentrifugiert und in 50 mM HEPES/TRIS (pH 6,8) suspendiert und auf eine OD₆₀₀ von 50 ±10 % eingestellt und auf Eis gelagert. Zur Kontaktierung mit einer der Verbindungen wurde eine Verbindung einer Stufe einer Verdünnungsreihe in DMSO in einem Reaktionsgefäß vorgelegt und darauf jeweils 80 µl der Hefesuspension pipettiert. Nach einer Inkubation auf Eis für 15 bis 20 min wurden 20 µl 5 mM Na-L-Laktat plus 0,04 µCi radioaktiv markiertes L-[1-¹⁴C]-Laktat zugegeben. Die erhaltene Laktat-Konzentration betrug 1 mM. Nach einer Inkubation von 30 s wurde die Aufnahme von Laktat durch schnelles Verdünnen mittels Zugabe von 1 ml eiskaltem Wasser gestoppt. Aus der so verdünnten Hefesuspension wurden die Hefezellen durch Vakuumfiltration auf eine Filtermembran gebracht, mit 7 ml kaltem Wasser gewaschen und mit der Filtermembran in 3 ml Szintillationscocktail überführt. Nach 24 h Inkubation bei 18°C in dem Szintillationscocktail, in dem die Zellen lysiert wurden, wurde die Menge radioaktiv markierten Laktats mittels eines Szintillationszählers gemessen. Aus der gemessenen Menge radioaktiv markierten Laktats wurde die von der Hefe aufgenommene Gesamtmenge Laktat berechnet. Als Positivkontrolle wurde in Parallelansätzen die Hefe mit DMSO ohne eine der Verbindungen mitgeführt und als 100 % FNT-Aktivität bzw. 0 % Hemmung angesehen. In Ansätzen mit Verbindung wurde eine im Vergleich mit den parallelen Positivkontrollen geringere Menge aufgenommenen Laktats gemessen. Als Negativkontrolle wurde Hefe mitgeführt, in der die endogenen Gene für die Monocarboxylat-Transporter Jen1p und Ady2p deletiert waren, jedoch nicht der FNT aus Plasmodium exprimiert wurde. Diese Negativkontrolle wurde als 0 % FNT-Aktivität bzw.100 % Hemmung angesehen.

Die mit der Hefe bestimmten IC50-Werte für das Wildtyp-FNT und die FNT-Mutante zeigten dieselbe Verschiebung wie die IC50-Werte der Plasmodium-Kultur. Dies zeigt zum einen, dass die getesteten Verbindungen ihre Wirkung auf Plasmodium durch Wechselwirkung mit dem FNT entfalten, bzw. dass FNT das Zielmolekül dieser Verbindungen bei der Hemmung von Plasmodium ist. Zum anderen zeigt dieses Ergebnis, dass die den FNT aus Plasmodium exprimierende Hefe stellvertretend für Plasmodium selbst in Verfahren zur Analyse der Hemmwirkung von Verbindungen auf Plasmodium eingesetzt werden kann. Die Ausbildung der Hemiketal-Prodrugform wirkt sich generell positiv auf die Wirksamkeit in Plasmodien-Kultur aus, während die Hefe die offenkettige Form ebenso gut aufnimmt.

### Beispiel 2: Herstellung von Verbindungen

Die Herstellung des Grundkörpers der Verbindungen kann durch Synthese mit den folgenden Schritten erfolgen: 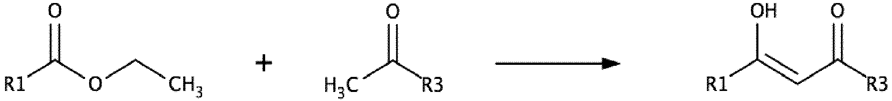 mit R1 = Perfluor-C₁- bis C₄-alkyl, R3 einer von

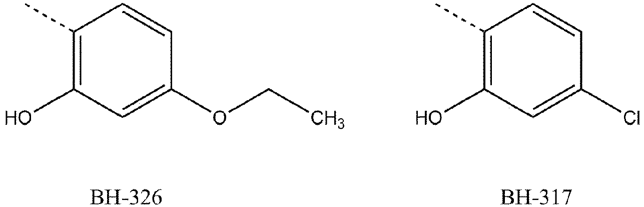

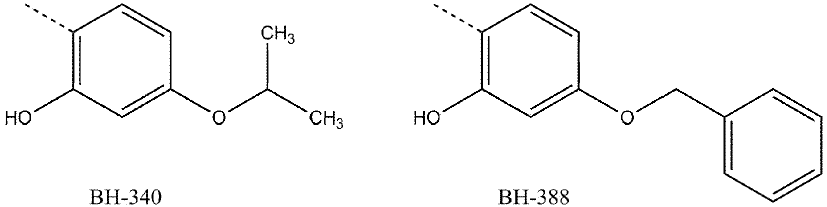

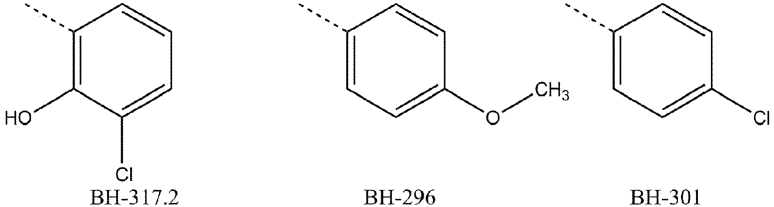

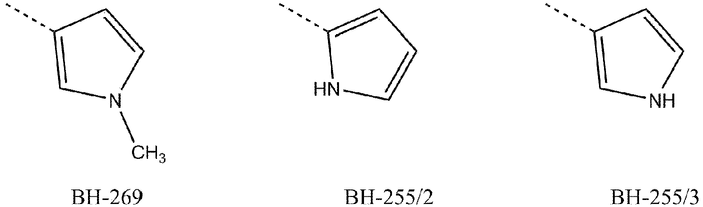

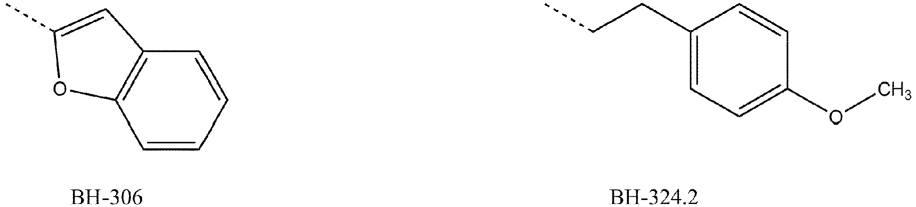

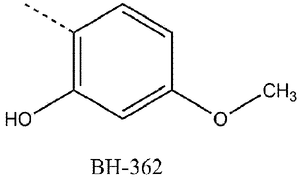

Für die Synthese werden 7,5 ml wasserfreies Tetrahydrofuran (THF) und 0,34 g (42,8 mmol) fein dispergiertes Lithiumhydrid in einem getrockneten 100 ml-Dreihalskolben vorgelegt, mit einem Rückflusskühler und einem Tropftrichter versehen, gerührt und zum Sieden erhitzt. Zu dieser Suspension wird ein Gemisch aus 15,0 mmol Perfluoralkylcarbonsäure-Ethylester, dessen Perfluoralkylgruppe R1 entspricht, und 12,5 mmol des mit R3 substituierten Ketons, gelöst in getrocknetem THF, langsam zugetropft. Das Reaktionsgemisch wird 3 h unter Rückfluss gekocht. Anschließend wird das THF am Rotationsverdampfer entfernt und der erhaltene Rückstand wird mit 60 ml einer kalten Mischung aus Essigsäure und Wasser (7:50) versetzt und das Gemisch mit 2 x 100 ml Ethylacetat extrahiert. Die vereinigten Phasen werden über Natriumsulfat getrocknet, einrotiert, über Kieselgel säulenchromatographisch aufgereinigt (Cyclohexan/Ethylacetat 90:10) und in n-Hexan umkristallisiert. Die Struktur der Verbindungen wird mittels 1H-NMR, 13C-NMR, 19F-NMR und Massenspektrometrie (LC-MS ESI) bestätigt. Die Veresterung der erhaltenen vinylogen Carbonsäure gemäß 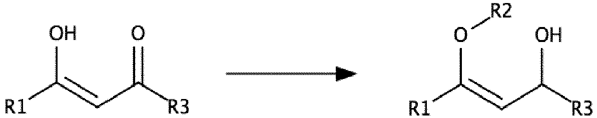 erfolgte durch Vorlegen von 5,0 ml wasserfreiem Dimethylformamid (DMF) mit 2 mmol der vinylogen Carbonsäureverbindung und 2 mmol (652 mg) Cäsiumcarbonat in einem getrockneten 100 ml-Dreihalskolben, mit Rückflusskühler und Tropftrichter versehen, und Rühren für 1 h bei 70°C. Zu der Suspension werden 2,2 mmol (442 mg) p-Toluolsulfonsäureethylester, gelöst in 5 ml getrocknetem DMF, langsam zugetropft und 6 h bei 70 °C gerührt. Das Gemisch wird auf Wasser gegeben, 2 x mit Diethylether extrahiert und die organischen Phasen über Natriumsulfat getrocknet.

Nach dem Einengen des Ethers am Rotationsverdampfer wird das erhaltene Rohprodukt über Kieselgel säulenchromatographisch aufgereinigt (Cyclohexan/Ethylacetat/Diethylamin 94:5:1). Die Struktur der Verbindungen wird mittels 1H-NMR, 13C-NMR, 19F-NMR und Massenspektrometrie (LC-MS ESI) bestätigt.

### Beispiel 3: Wirkung von Verbindungen gegen FNT

Stellvertretend für ein Formiat-Nitrit-Transporterprotein (FNT) aus Protozoen wurde das FNT von Plasmodium falciparum als Wildtyp und als Gly107Ser-Mutante verwendet, die jeweils in Hefe exprimiert und mit Verdünnungen von zu testenden Verbindungen inkubiert wurden, wie in Beispiel 1 beschrieben.

### Dabei wurden die Verbindungen

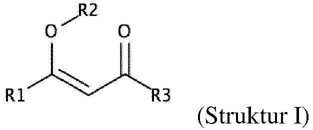

mit
R1 = Pentafluorethyl oder Heptafluorpropyl (siehe BH-362), R2 = H und
R3 einer der Reste 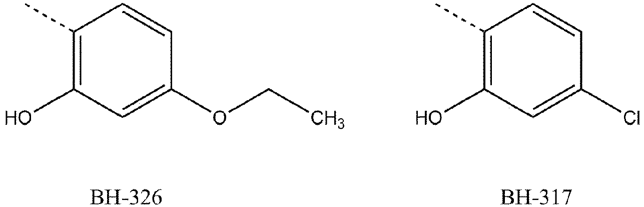 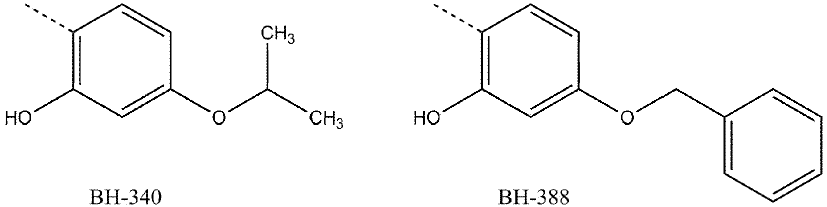 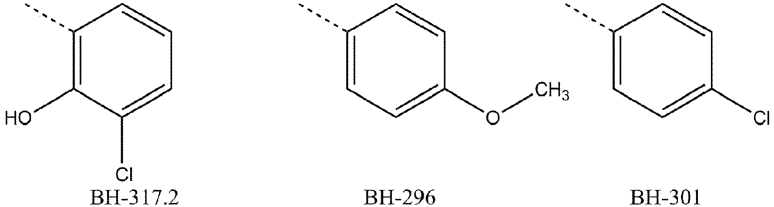 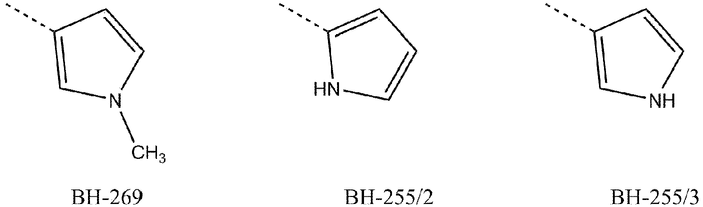 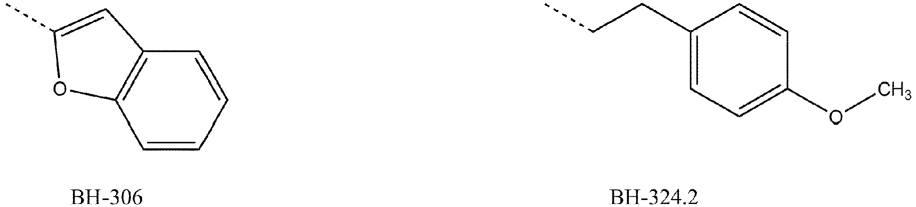 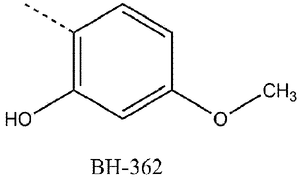 eingesetzt, die jeweils nach der Bezeichnung benannt wurden, der unter R3 angegeben ist. Die folgenden Hemmwerte wurden erhalten:

| | Hefe- Testsystem | | Plasmodium-Kultur | |
|---|---|---|---|---|
| R1 = Pentafluorethyl; | IC50 gegen FNT Wildtyp (µM) | IC50 gegen Gly107Ser-Mutante von FNT (µM) | IC50 gegen Wildtyp (µm) | IC50 gegen Gly107Ser-Mutante (µM) |
| R2 = H | | | | |
| BH-326 | 0,24 | nicht bestimmt | 0,05 | nicht bestimmt |
| BH-317 | 0,12 | nicht bestimmt | 0,37 | nicht bestimmt |
| BH-340 | 0,40 | nicht bestimmt | 0,10 | nicht bestimmt |
| BH-388 | 0,89 | nicht bestimmt | nicht bestimmt | nicht bestimmt |
| BH-317.2 | 0,25 | nicht bestimmt | nicht bestimmt | nicht bestimmt |
| BH-296 | 0,14 | 2,7 | 3,4 | 8,9 |
| BH-301 | 0,12 | 5,8 | nicht bestimmt | nicht bestimmt |
| BH-269 | 0,13 | 15 | nicht bestimmt | nicht bestimmt |
| BH-255/2 | 0,16 | 12 | nicht bestimmt | nicht bestimmt |
| BH-255/3 | 0,20 | 13 | nicht bestimmt | nicht bestimmt |
| BH-306 | 0,18 | 15 | nicht bestimmt | nicht bestimmt |
| BH-324.2 | 0,16 | 6,3 | nicht bestimmt | nicht bestimmt |
| | | | | |
| R1 = Heptafluorpropyl; | IC50 gegen FNT Wildtyp (µM) | IC50 gegen Gly107Ser-Mutante von FNT (µM) | IC50 gegen Wildtyp (µm) | IC50 gegen Gly107Ser-Mutante (µM) |
| R2 = H | | | | |
| BH-362 | 0,25 | nicht bestimmt | 0,57 | nicht bestimmt |

Die IC50-Werte zeigen, dass die getesteten neuen Verbindungen den FNT von Plasmodium signifikant hemmen. Aus diesen Ergebnissen geht auch hervor, dass insbesondere BH-326, BH-340 und B-317 sowohl eine effektive Hemmung der FNT bewirken, wie im Hefe-Testsystem gezeigt ist, als auch eine effektive Hemmung der Plasmodium-Kultur bewirken. Dabei weisen diese Verbindungen als R3 eine Phenylgruppe auf, die in ortho-Stellung zur Ketogruppe eine Hydroxylgruppe aufweist und in BH-317 in para-Stellung zur Ketogruppe ein Cl als Halogenatom, bzw. BH-326 und BH-340 einen Alkoxyrest in para-Stellung zur Ketogruppe aufweist, wobei der Alkoxyrest bei BH-326 eine Ethoxygruppe ist und in BH-340 eine Isopropoxygruppe.

Weiterhin zeigen die IC50-Werte, dass bevorzugt die Verbindungen BH-296, BH-301 und BH-324.2 auch die Gly107Ser-Mutante signifikant hemmen.

Die Verbindungen zeigen eine Abhängigkeit der IC50 von der Zeit der Vorinkubation, bevor Laktat zu den Zellen gegeben wird. Dabei zeigt sich, dass eine längere Vorinkubation zu einer erhöhten Transporthemmung führt. Bei Verlängerung der Vorinkubation mit MMV007839 auf 24 h vor der Zugabe von Laktat wird ein IC50 von 15 nM anstelle des IC50 von 170 nM nach 20 min bestimmt. Die Hemmung des FNT konnte nicht durch Waschen der Zellen mit 50 mM HEPES-Tris, pH6,8 verringert werden und die Hemmung blieb über Stunden erhalten. Daher wird angenommen, dass die Verbindung jedenfalls durch Waschen nicht vom FNT entfernt wird. Dieses Ergebnis deutet darauf hin, dass die Verbindungen irreversibel an den FNT binden, bzw. die Verbindungen eine Dauerblockade des FNT durch Suizidhemmung verursachen. Es wird vermutet, dass die Verbindungen die Struktur zweier Laktat-Moleküle nachahmen, eines in der anionischen Form (vinyloge Carbonsäure der Struktur I) und eines in der neutralen Milchsäureform (Fluoralkyl-Rest, R1).

Daher wird gegenwärtig angenommen, dass das Pharmakophor von Struktur I mit dem Perfluor-C₂- bis C₄-alkyl als R1 gebildet wird und der Rest R3 variiert werden kann, z.B. sterisch deutlich über einen aromatischen Rest R3 erweitert werden kann. Das Fluoralkyl R1 kann in den lipophilen Transportkanal des FNT ragen, während die negativ geladene Struktur I elektrostatisch mit dem Transportereingang des FNT interagiert.

## Patentansprüche

1. Verbindung zur Verwendung bei der Behandlung von Infektionen durch Protozoen, die ein Formiat-Nitrit-Transporterprotein (FNT) aufweisen, die die Struktur I aufweist, in der der Rest R1 ein Perfluoralkyl ist, bei dem das Alkyl ein gradkettiges oder verzweigtes ist 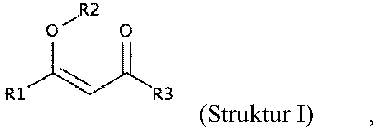 wobei R2 H oder ein C₁- bis C₁₂-Alkyl ist oder R2 eine Carbonylgruppe mit H oder einem C₁- bis C₁₂-Alkyl ist, R1 Pentafluorethyl, Heptafluorpropyl oder Nonafluorbutyl ist, R3 ein Aromat ist, der direkt oder über ein C₁ bis C₃-Alkyl gebunden ist, und der in para- oder meta-Stellung zur Struktur I mit einem gradkettigen oder verzweigten C₁- bis C₁₂-Alkylrest oder einem C₁- bis C₁₂-Alkoxyrest oder Halogen substituiert ist.

2. Verbindung zur Verwendung bei der Behandlung von Infektionen durch Protozoen nach Anspruch 1, **dadurch gekennzeichnet, dass** die Verwendung zur Behandlung einer Infektion eines Menschen oder Tiers mit Protozoen ist.

3. Verbindung zur Verwendung bei der Behandlung von Infektionen durch Protozoen nach einem der voranstehenden Ansprüche, **dadurch gekennzeichnet, dass** R3 ein aromatischer 5-Ring oder ein aromatischer 6-Ring ist.

4. Verbindung zur Verwendung bei der Behandlung von Infektionen durch Protozoen nach einem der voranstehenden Ansprüche, **dadurch gekennzeichnet, dass** R3 ein Phenyl, ein Pyrrolyl, Furyl, Pyridyl oder Benzofuryl ist.

5. Verbindung zur Verwendung bei der Behandlung von Infektionen durch Protozoen nach einem der Ansprüche 1 bis 4, **dadurch gekennzeichnet, dass** das C₁- bis C₁₂-Alkyl von R2 und/oder der C₁- bis C₁₂-Alkylrest von R3 unabhängig voneinander Methyl, Ethyl, *n*-Propyl, *n*-Butyl, *n*-Pentyl, *n*-Hexyl, *n*-Octyl, *n*-Decyl, *n*-Dodecyl, *iso-*Propyl, *iso*-Butyl, tert-Butyl, 2,2-Dimethylpropyl oder Cyclohexyl ist.

6. Verbindung zur Verwendung bei der Behandlung von Infektionen durch Protozoen nach einem der voranstehenden Ansprüche, **dadurch gekennzeichnet, dass** R3 eine Hydroxylgruppe im Abstand von zwei Kohlenstoffen zum Carbonyl-C-Atom der Struktur I zur Ausbildung des Hemiketal-Prodrugs enthält.

7. Verbindung zur Verwendung bei der Behandlung von Infektionen durch Protozoen nach einem der voranstehenden Ansprüche, **dadurch gekennzeichnet, dass** R3 einen Phenylring aufweist, der in ortho-Stellung zum Carbonyl-C-Atom eine Hydroxylgruppe aufweist und in para-Stellung zum Carbonyl-C-Atom einen Alkoxyrest.

8. Verbindung zur Verwendung bei der Behandlung von Infektionen durch Protozoen nach Anspruch 6 oder 7, **dadurch gekennzeichnet, dass** der Alkoxyrest eine Methoxygruppe, eine Ethoxygruppe oder eine Propoxygruppe ist.

9. Verbindung zur Verwendung bei der Behandlung von Infektionen durch Protozoen nach einem der voranstehenden Ansprüche, **dadurch gekennzeichnet, dass** R3 unter 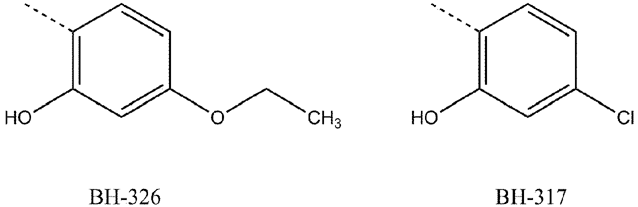 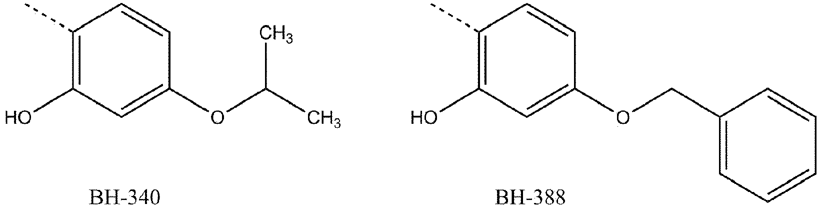 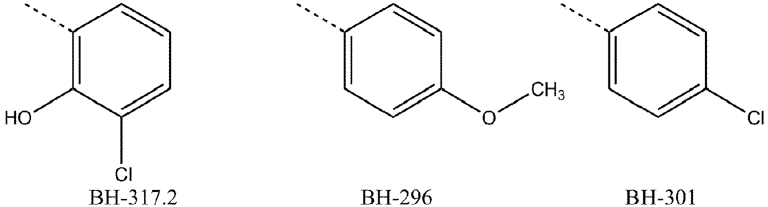 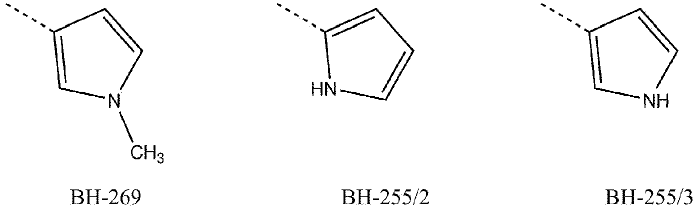 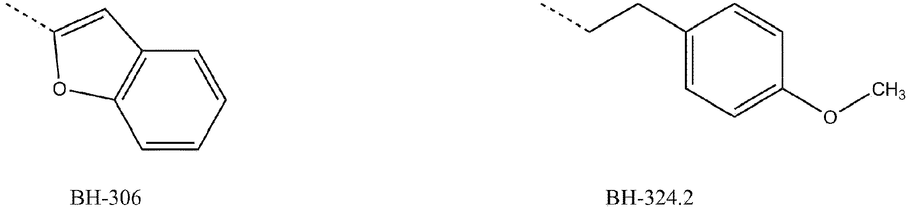 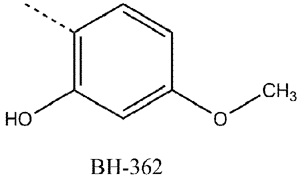 ausgewählt ist.

10. Verbindung zur Verwendung bei der Behandlung von Infektionen durch Protozoen nach einem der voranstehenden Ansprüche, **dadurch gekennzeichnet, dass** die Protozoen das Formiat-Nitrit-Transporterprotein von Plasmodium falciparum, von Plasmodium vivax, von Toxoplasma gondii oder Entamoeba histolytica oder dessen Gly107Ser-Mutante aufweisen.

## Claims

1. Compound for use in the treatment of infections by protozoa having a formate-nitrite transporter protein (FNT), the compound having structure I, wherein R1 is a perfluoroalkyl in which the alkyl is straight-chain or branched 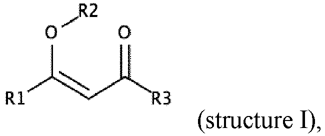 wherein R2 is H or a C₁ to C₁₂ alkyl, or R2 is a carbonyl group with H or a C₁ to C₁₂ alkyl, wherein R1 is pentafluoroethyl, heptafluoropropyl or nonafluorobutyl, wherein R3 is an aromatic compound which is bonded directly or by a C₁ to C₃ alkyl and which is substituted in the para or meta position to structure I by a straight-chain or branched C₁ to C₁₂ alkyl group or a C₁ to C₁₂ alkoxy group or halogen.

2. Compound for use in the treatment of infections by protozoa according to claim 1, **characterized in that** the use is for the treatment of an infection of a human or animal by protozoa.

3. Compound for use in the treatment of infections by protozoa according to one of the preceding claims, **characterized in that** R3 is an aromatic 5-membered ring or an aromatic 6-membered ring.

4. Compound for use in the treatment of infections by protozoa according to one of the preceding claims, **characterized in that** R3 is a phenyl, a pyrrolyl, furyl, pyridyl or benzofuryl.

5. Compound for use in the treatment of infections by protozoa according to one of claims 1 to 4, **characterized in that** the C₁ to C₁₂ alkyl of R2 and/or the C₁ to C₁₂ alkyl group of R3 is independently methyl, ethyl, *n*-propyl, *n*-butyl, *n*-pentyl, *n*-hexyl, *n-*octyl, *n*-decyl, *n*-dodecyl, *iso*-propyl, *iso*-butyl, *tert*-butyl, 2,2-dimethylpropyl or cyclohexyl.

6. Compound for use in the treatment of infections by protozoa according to one of the preceding claims, **characterized in that** R3 contains a hydroxyl group at a distance of two carbons from the carbonyl C atom of structure I to form the hemiketal prodrug.

7. Compound for use in the treatment of infections by protozoa according to one of the preceding claims, **characterized in that** R3 comprises a phenyl ring which has a hydroxyl group in ortho-position to the carbonyl C atom and has an alkoxy group in para-position to the carbonyl C atom.

8. Compound for use in the treatment of infections by protozoa according to claim 6 or 7, **characterized in that** the alkoxy group is a methoxy group, an ethoxy group or a propoxy group.

9. Compound for use in the treatment of infections by protozoa according to one of the preceding claims, **characterized in that** R3 is selected from

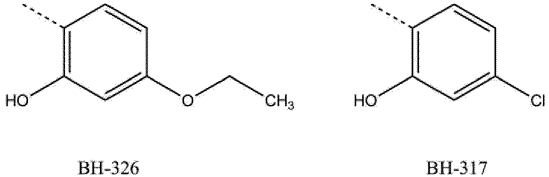

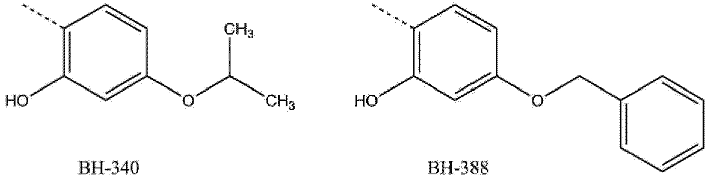

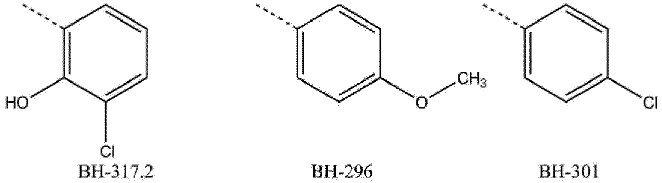

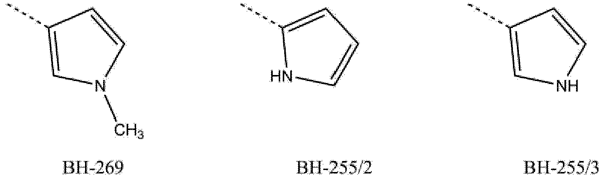

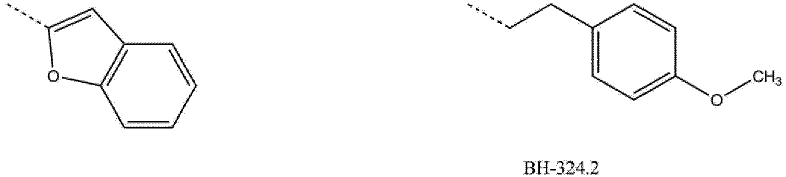

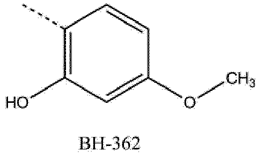

10. Compound for use in the treatment of infections by protozoa according to one of the preceding claims, **characterized in that** the protozoa comprise the formate-nitrite transporter protein of Plasmodium falciparum, of Plasmodium vivax, of Toxoplasma gondii, or of Entamoeba histolytica, or the Gly107Ser mutant thereof.

## Revendications

1. Composé à utiliser dans le traitement d'infections par des protozoaires ayant une protéine de transporteur de formate-nitrite (FNT), le composé ayant la structure I dans laquelle R1 est un perfluoroalkyle dans lequel l'alkyle est à chaîne linéaire ou ramifiée 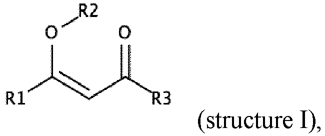 R2 est H ou un alkyle en C₁ à C₁₂, ou R2 est un groupe carbonyle avec H ou un alkyle en C₁ à C₁₂, R1 est un pentafluoroéthyle, un heptafluoropropyle ou un nonafluorobutyle, R3 est un aromatique qui est lié directement ou par l'intermédiaire d'un alkyle en C₁ à C₃ et qui est substitué en position para ou méta à la structure I par un groupe alkyle en C₁ à C₁₂ linéaire ou ramifié ou un groupe alcoxy en C₁ à C₁₂ ou un halogène.

2. Composé à utiliser dans le traitement d'infections par des protozoaires selon la revendication 1, **caractérisé en ce que** l'utilisation est destinée à traiter une infection d'un humain ou d'un animal par des protozoaires.

3. Composé à utiliser dans le traitement d'infections par des protozoaires selon l'une des revendications précédentes, **caractérisé en ce que** R3 est un cycle aromatique à 5 chaînons ou un cycle aromatique à 6 chaînons.

4. Composé à utiliser dans le traitement d'infections par des protozoaires selon l'une des revendications précédentes, **caractérisé en ce que** R3 est un phényle, un pyrrolyle, un furyle, un pyridyle ou un benzofuryle.

5. Composé à utiliser dans le traitement d'infections par des protozoaires selon l'une des revendications 1 à 4, **caractérisé en ce que** l'alkyle en C₁ à C₁₂ de R2 et/ou l'alkyle en C₁ à C₁₂ de R3 est indépendamment un méthyle, un éthyle, un *n-*propyle, un *n*-butyle, un *n*-pentyle, un *n*-hexyle, un *n*-octyle, un *n*-décyle, un *n-*dodécyle, un *iso*-propyle, un *iso*-butyle, un *tert*-butyle, un 2,2-diméthylpropyle ou un cyclohexyle.

6. Composé à utiliser dans le traitement d'infections par des protozoaires selon l'une des revendications précédentes, **caractérisé en ce que** R3 contient un groupe hydroxyle espacé de deux carbones de l'atome par rapport à l'atome de carbone carbonyle de la structure I pour former le promédicament hémiketal.

7. Composé à utiliser dans le traitement d'infections par des protozoaires selon l'une des revendications précédentes, **caractérisé en ce que** R3 comprend un cycle phényle ayant un groupe hydroxyle en ortho par rapport à l'atome de carbone carbonyle et un groupe alcoxy en para par rapport à l'atome de carbone carbonyle.

8. Composé à utiliser dans le traitement d'infections par des protozoaires selon la revendication 6 ou 7, **caractérisé en ce que** le groupe alcoxy est un groupe méthoxy, un groupe éthoxy ou un groupe propoxy.

9. Composé à utiliser dans le traitement d'infections par des protozoaires selon l'une des revendications précédentes, **caractérisé en ce que** R3 est choisi parmi

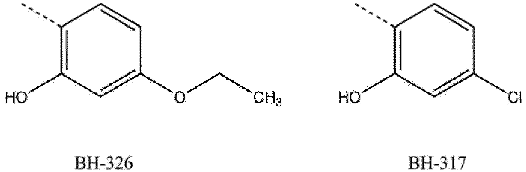

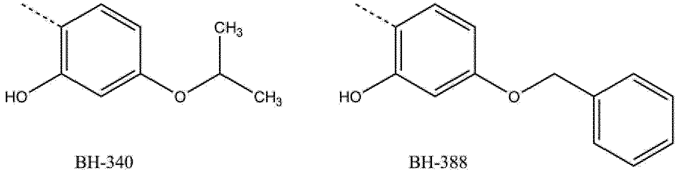

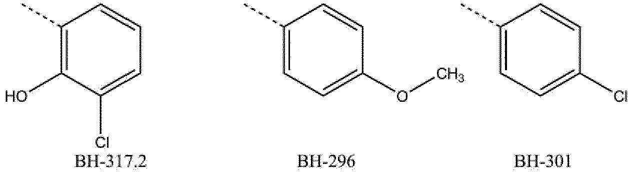

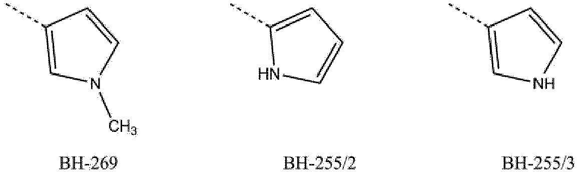

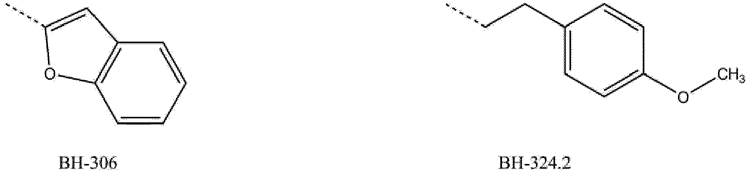

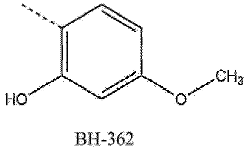

10. Composé à utiliser dans le traitement d'infections par des protozoaires selon l'une des revendications précédentes, **caractérisé en ce que** les protozoaires comprennent la protéine transporteur formate-nitrite de Plasmodium falciparum, de Plasmodium vivax, de Toxoplasma gondii ou d'Entamoeba histolytica ou de son mutant Gly107Ser.
